Europäisches Patentamt

European Patent Office

Office européen des brevets

(11) Numéro de publication: **0 290 307 B1**

(12) **FASCICULE DE BREVET EUROPEEN**

(45) Date de publication de fascicule du brevet: **08.07.92** (51) Int. Cl.⁵: **A61F 6/06**

(21) Numéro de dépôt: **88400934.1**

(22) Date de dépôt: **18.04.88**

(54) **Tampon en éponge élastique, notamment tampon vaginal.**

(30) Priorité: **29.04.87 FR 8706124**

(43) Date de publication de la demande:
**09.11.88 Bulletin 88/45**

(45) Mention de la délivrance du brevet:
**08.07.92 Bulletin 92/28**

(84) Etats contractants désignés:
**AT BE CH DE ES FR GB GR IT LI LU NL SE**

(56) Documents cités:
**EP-A- 29 708**       **EP-A- 0 084 755**
**DE-C- 822 877**      **DE-C- 828 141**
**FR-A- 2 463 609**    **US-A- 4 564 362**

(73) Titulaire: **Augros, Jacques**
**Avenue de la Croix Baillet**
**F-95400 Villiers le Bel(FR)**

(72) Inventeur: **Augros, Jacques**
**Avenue de la Croix Baillet**
**F-95400 Villiers le Bel(FR)**

(74) Mandataire: **Bertrand, Didier et al**
**Cabinet Beau de Loménie 55, rue d'Amster-dam**
**F-75008 Paris(FR)**

Rank Xerox (UK) Business Services

## Description

L'invention concerne un tampon constitué d'un corps en éponge élastique, en mousse, ou analogue.

L'utilisation, notamment à des fins contraceptives, de tampons vaginaux ronds ou aplatis faits d'éponge, mousse ou autre substance absorbante élastique, imprégnée de produits à action spermicide ou médicamenteuse est connue depuis de nombreuses années.

Si l'introduction manuelle de ces tampons est relativement aisée, leur extraction après utilisation est souvent difficile et déplaisante pour la femme lorsqu'il n'est pas prévu de dispositif d'extraction. Aussi des tampons connus comportent-ils un dispositif d'accrochage digital rapporté, tel qu'une boucle (cf. EP-A-29708), ou un fil (cf. FR-A-2 463 609) permettant de saisir le tampon par le doigt et de le retirer. Mais ce dispositif d'accrochage, extérieur au corps du tampon, est ressenti comme un inconvénient pour le couple, du fait de sa présence et de sa dureté : il est souvent perçu et peut provoquer des irritations de part et d'autre. Cela limite fortement le développement de cette méthode de contraception.

L'objet de l'invention a pour but de remédier à ces inconvénients et de proposer un tampon permettant un accrochage et une extraction facile après utilisation.

L'invention atteint son but au moyen d'un tampon comportant un corps en matière élastique et un dispositif d'accrochage digital en vue de la mise en place ou de l'extraction du tampon, réalisé par au moins un évidement du corps du tampon, vois DE-C-828 141, caractérisé en ce que le corps est en mousse et de forme générale sensiblement cylindrique aplatie et l'évidement est situé marginalement, sensiblement parallèlement à un bord du corps et possède au moins un bord en décrochement sensiblement perpendiculaire à une surface contiguë du corps du tampon pour permettre au doigt de prendre appui sur ce bord afin d'entraîner le tampon, en vue essentiellement de son introduction, ou surtout de son extraction.

Un autre objet de l'invention est un procédé de fabrication du tampon précédent caractérisé en ce que l'évidement est obtenu par une opération unique de découpe, de préférence simultanée à l'opération de découpe du tampon lui-même.

On comprendra que l'invention n'a rien à voir avec les cavités ou orifices qui sont parfois prévus dans les pessaires. D'une part ces pessaires sont généralement faits dans un matériau autre qu'une mousse imprégnable (DE-C-828 141 ou EP-A-84 755) et les problèmes rencontrés sont donc d'une nature différente de ceux de l'invention. D'autre part, les orifices n'ont pas pour but de constituer des dispositifs d'accrochage du doigt ; dans les dispositifs en éponge connus, même si des évidements peuvent être prévus pour différentes fonctions, ils n'excluent pas la présence de dispositifs d'accrochage supplémentaires (DE-C-822 877 ou US-A-4 564 362).

L'évidement conforme à l'invention est crée par une séparation localisée dans la texture du produit, obtenue par découpe, usinage, moulage, à une étape quelconque de la fabrication du tampon. De façon préférentielle, l'évidement est formé par découpe, à l'emporte-pièce ou à l'aide d'une matrice de découpage par exemple, avantageusement en même temps que le tampon lui-même, à partir d'un flan primitif. Cet évidement est de préférence constitué par une entaille allongée et de faible épaisseur, traversante ou non. Dans sa forme la plus avantageuse, l'évidement est une entaille d'épaisseur nulle ou sensiblement nulle, obtenue par simple incision ou matriçage du tampon de mousse.

L'évidement est excentré, situé marginalement et sensiblement parallélement à un bord du corps.

Naturellement, on peut avoir un ou plusieurs évidements : en particulier, il est avantageux d'avoir deux évidements respectivement pour la mise en place et l'extraction du tampon. Ces évidements sont de préférence dans deux zones opposées du tampon de sorte que l'introduction du tampon grâce au premier évidement permet de positioner en même temps l'évidement opposé dans la position basse qui facilitera son accrochage par l'index au moment du retrait.

Les évidements peuvent être en arc de cercle, ou avoir des formes et des orientations différentes, fonctions des buts recherchés, et de la configuration et du volume du tampon.

Celui-ci a généralement une forme de disque plat.

Du fait que les évidements sont pratiqués dans le corps du tampon lui-même, il n'est donc introduit aucun corps dur étranger au tampon. De plus, le coût de fabrication de ces évidements est très réduit.

Un introducteur-extracteur peut bien entendu être utilisé pour la pose et le retrait, son utilisation et son efficacité étant renforcées par la présence des évidements conformes à l'invention.

Celle-ci s'applique d'ailleurs aussi à d'autres utilisations que la contraception ou la prévention des maladies sexuellement transmissibles, notamment le sida. En dehors de la santé, l'invention peut concerner des tampons pour produits industriels, d'entretien, ménagers, cosmétologiques, ou toutes autres applications nécessitant une préhension ou une tenue.

D'autres caractéristiques et avantages de l'invention ressortiront de la description suivantes de plusieurs modes de réalisation. Il sera fait référence aux dessins annexés sur lesquels :

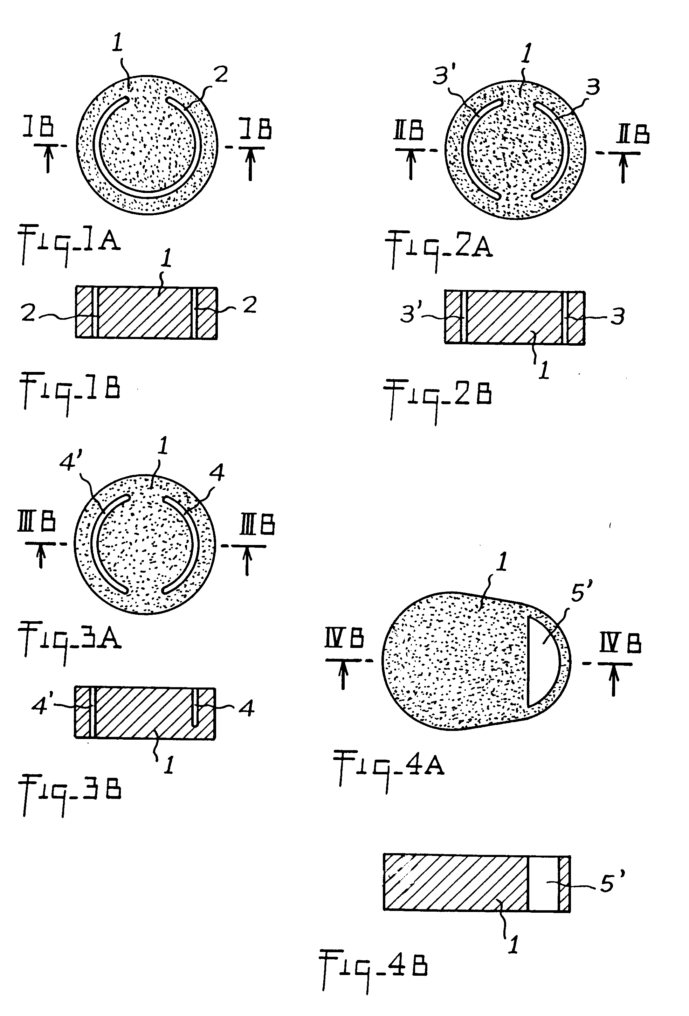

. les figures 1A et 1B montrent en plan et en coupe I-I un premier mode de réalisation,

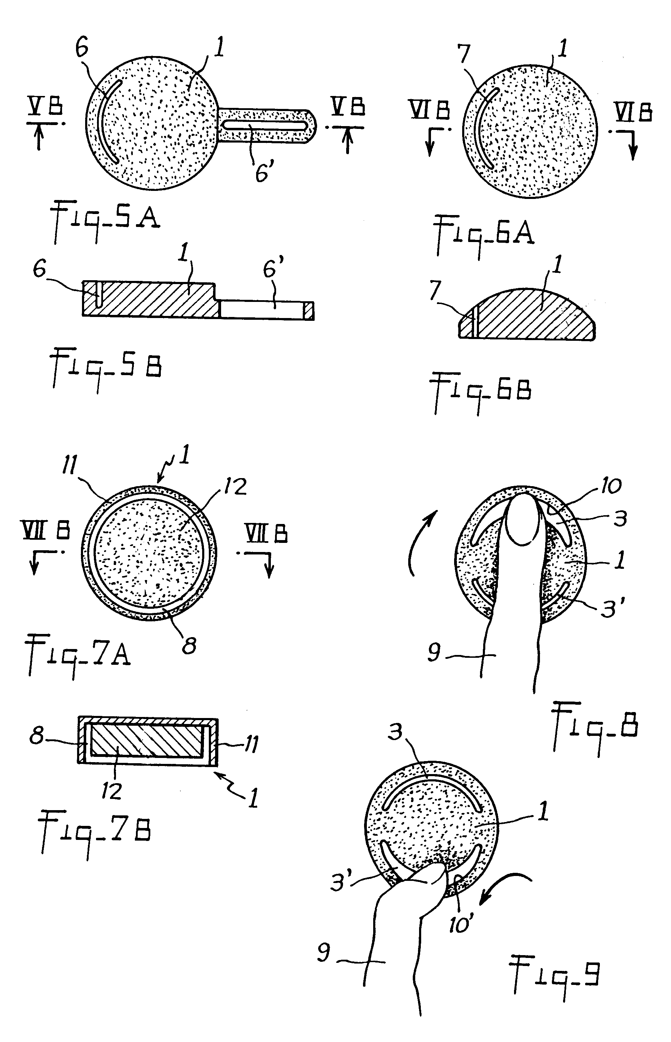

. les figures 2A et 2B montrent en plan et en coupe II-II un deuxième mode de réalisation,

. les figures 3A et 3B montrent en plan et en coupe III-III un troisième mode de réalisation,

. les figures 4A et 4B montrent en plan et en coupe IV-IV un quatrième mode de réalisation,

. les figures 5A et 5B montrent en plan et en coupe V-V un cinquième mode de réalisation,

. les figures 6A et 6B montrent en plan et en coupe VI-VI un sixième mode de réalisation,

. les figures 7A et 7B montrent en plan et en coupe VII-VII un septième mode de réalisation,

. les figures 8 et 9 illustrent le procédé de mise en place et d'extraction du tampon de l'invention.

Le tampon comporte un corps 1 en éponge souple élastique imprégnable réalisée dans une mousse à pores ouverts. Dans ce corps 1 est pratiqué un évidement 2 à 4, 6 à 8, 3' à 6' (figs.1 à 7) dans lequel le doigt 9 (figs.8,9) peut s'introduire partiellement pour avoir prise sur l'une des parois latérales 10,10′ de l'évidement 3,3′ en vue de la mise en place (fig.8) ou de l'extraction (fig.9) du tampon, et ce grâce à la déformabilité du matériau constituant le corps 1.

L'évidement peut être relativement étroit et allongé (comme représenté sur l'ensemble des dessins), présenter donc un rapport longueur / largeur élevé. En fait, les évidements des figures 1,2 et 3 par exemple sont de préférence de simples coupures d'épaisseur nulle (on a représenté une épaisseur uniquement pour la clarté du dessin) obtenue par une opération unique d'incision dans le tampon. Cette opération peut être simultanée avec l'opération de découpe générale du tampon.

Le tampon est de forme générale cylindrique aplatie. Le cylindre est à base circulaire (figs.1,2,3,7) ou non : ainsi une base trapézoïdale arrondie (fig.4) ou circulaire avec appendice (fig.5) conviennent aussi.

Une ou les bases du cylindres peuvent être plates (figs.1,2,3,4), réparties sur plusieurs plans (fig.5) ou gauche (figure 6).

Les évidements 2,3,3',4,5',6',7 traversent l'épaisseur du tampon, tandis que les évidements 4,6,8, sont borgnes.

L'avantage des évidements traversants est qu'ils séparent complètement le corps du tampon à leur niveau, et permettent donc une plus grande déformation et un plus grand écartement d'au moins l'une des lèvres de l'évidement (figs.8,9). Lorsque l'évidement traversant est pratiqué le long d'un bord du tampon, il se forme une boucle de traction facilitant la préhension du tampon (figs.8,9). Le fait de tirer le tampon par l'intermédiaire d'une boucle évite, comme il est nécessaire avec les autres dispositifs, de pincer et pousser le tampon, lui faisant rejeter une quantité non négligeable du produit dont il est imprégné, ce qui constitue une perte du produit actif et une gêne pour l'utilisatrice.

L'avantage des évidements borgnes en revanche est qu'ils sont plus résistants, ou peuvent être en coutour fermé (évidement 8, fig.7).

Naturellement, on peut combiner sur un même tampon des évidements traversants ou borgnes.

Lorsque deux évidements sont prévus, il est avantageux qu'ils intéressent deux zones opposées du tampon (figs.2,3,4,5,6, 8,9) de manière à pouvoir être utilisées respectivement pour l'insertion (fig.8) et l'extraction (fig.9) du tampon par le doigt 9. Les évidements sont de préférence situés au bord des zones afin que la plus grande partie du corps 1 du tampon soit entraînée en traction.

Il est possible d'obtenir le même effet avec un évidement unique placé dans deux zones opposées, de préférence marginalement (fig.1, évidement 2) ou au centre (fig.7, évidement 8).

Les évidements sont créés directement dans le corps du tampon par simple découpe, sciage, usinage ou tout autre procédé.

Les tampons peuvent, les cas échéant, comprendre des pièces assemblées, comme montré sur les figures 7A,7B, où le corps 1 est formé d'une première partie 11 en forme de cuvette à bord cylindrique au fond de laquelle est rapportée une seconde partie 12 en forme de disque. L'évidement 8 est formée par l'espace laissé entre le bord interne de la cuvette 11 et le bord extérieur du disque 12. Les pièces assemblées peuvent présenter des caractéristiques différentes de dureté, pouvoir d'absorption, d'isolation, de diffusion, d'état de surface, etc. Elles peuvent éventuellement être imprégnées de produits différents ou incompatibles : produits réactifs entre eux (moussants), à actions complémentaires, micro-encapsulés.

**Revendications**

1. Tampon, notamment tampon vaginal, comportant un corps (1) en matière élastique et un dispositif d'accrochage digital en vue de la mise en place ou de l'extraction du tampon, réalisé par au moins un évidement (2,3,4,6,7,8,3',4',5',6') du corps du tampon, caractérisé en ce que le corps (1) est en mousse et de forme générale sensiblement cylindrique aplatie, l'évidement (2-8) est situé marginalement et sensiblement parallèlement à un bord du corps (1) et possède au moins un bord en décrochement sensiblement perpendiculaire à une surface contiguë du corps du tampon, formant appui par un doigt.

2. Tampon selon la revendication 1, caractérisé en ce que l'évidement (2,3,3',4',5',6',7) est traversant.

3. Tampon selon la revendication 1, caractérisé en ce que l'évidement (4,6,8) est borgne.

4. Tampon selon l'une quelconque des revendications 1 à 3, caractérisé en ce que l'évidement est une entaille allongée.

5. Tampon selon l'une quelconque des revendications 1 à 4, caractérisé en ce que l'évidement est à la forme d'une courbe.

6. Tampon selon l'une quelconque des revendications 1 à 5, caractérisé en ce qu'il comporte deux évidements (3,3',4,4') prévus dans deux zones opposées du tampon.

7. Procédé de fabrication d'un tampon selon l'une quelconque des revendications 1 à 6, caractérisé en ce que l'évidement (2,3,3',4,4',5',6',7,8,) est obtenu par une opération unique de découpe, de préférence simultanée à l'opération de découpe du tampon lui-même.

## Claims

1. Pad, notably vaginal pad, comprising a body (1) in elastic material and a digital holding device for the purpose of inserting and extracting the pad, produced by at least one recess (2, 3, 4, 6, 7, 8, 3', 4', 5', 6') in the body of the pad, characterized in that the body (1) is in foam, and of substantially cylindrical flattened general shape, the recess (2-8) is situated marginally and substantially in parallel to an edge of the body (1) and has at least one set back edge substantially perpendicular to a contiguous surface of the body of the pad, forming a rest for one finger.

2. Pad according to claim 1, characterized in that the recess (2, 3, 3', 4', 5', 6', 7) is a through recess.

3. Pad according to claim 1, characterized in that the recess (4, 6, 8) is a blind recess.

4. Pad according to any one of claims 1 to 3, characterized in that the recess is an elongated slit.

5. Pad according to any one of claims 1 to 4, characterized in that the recess forms a curve.

6. Pad according to any one of claims 1 to 5, characterized in that it comprises two recesses (3, 3', 4, 4') provided in two opposite zones of the pad body.

7. Method for producing a pad according to any one of claims 1 to 6, characterized in that the recess (2, 3, 3', 4, 4', 5', 6, 6', 7, 8) is obtained by a single cutting operation, preferably simultaneously to the cutting operation of the pad itself.

## Patentansprüche

1. Tampon, insbesonders Vaginaltampon, umfassend einen Körper (1) aus elastischem Material und eine Fingerverankerungsvorrichtung für das Einführen und das Entnehmen des Tampons, gebildet aus zumindest einer Ausnehmung (2,3,4,6,7,8,3',4',5',6') im Tamponkörper, dadurch gekennzeichnet, daß der Körper (1) schwammartig ist und im wesentlichen eine allgemein zylindrische, abgeplattete Form hat, wobei die Ausnehmung (2-8) am Rand und im wesentlichen parallel zu einem Rand des Körpers (1) angeordnet ist und zumindest einen Entnahmerand besitzt, der im wesentlichen rechtwinkelig zu einer benachbarten Oberfläche des Tamponkörpers verläuft und eine Rast für einen Finger bildet.

2. Tampon nach Anspruch 1, dadurch gekennzeichnet, daß die Ausnehmung (2,3,3',4',5',6',7) durchgehend ist.

3. Tampon nach Anspruch 1, dadurch gekennzeichnet, daß die Ausnehmung (4,6,8) nicht durchgehend ist.

4. Tampon nach einem der Ansprüche 1 bis 3, dadurch gekennzeichnet, daß die Ausnehmung ein länglicher Einschnitt ist.

5. Tampon nach einem der Ansprüche 1 bis 4, dadurch gekennzeichnet, daß die Ausnehmung Kurvenform hat.

6. Tampon nach einem der Ansprüche 1 bis 5, dadurch gekennzeichnet, daß er zwei Ausnehmungen (3,3',4,4') aufweist.

7. Verfahren zur Herstellung eines Tampons nach einem der Ansprüche 1 bis 6, dadurch gekennzeichnet, daß die Ausnehmung (2,3,3',4,4',5',6,6',7,8) durch eine einzige Schneidoperation, vorzugsweise simultan mit dem Abschneiden des Tampons selbst, erhalten wird.

Fig. 1A

Fig. 1B

Fig. 2A

Fig. 2B

Fig. 3A

Fig. 3B

Fig. 4A

Fig. 4B

Fig. 5A

Fig. 5B

Fig. 6A

Fig. 6B

Fig. 7A

Fig. 7B

Fig. 8

Fig. 9